(19) Europäisches Patentamt
European Patent Office
Office européen des brevets

(11) Numéro de publication : **0 520 889 A1**

(12) **DEMANDE DE BREVET EUROPEEN**

(21) Numéro de dépôt : **92401789.0**

(22) Date de dépôt : **25.06.92**

(51) Int. Cl.[5] : **C08J 3/14,** C08G 63/66, A61K 9/51, // C08L67/04, C08L71/02

(30) Priorité : **28.06.91 FR 9108042**

(43) Date de publication de la demande : **30.12.92 Bulletin 92/53**

(84) Etats contractants désignés : **PT**

(71) Demandeur : **RHONE-POULENC RORER SA
20, avenue Raymond Aron
F-92160 Antony (FR)**

(72) Inventeur : **Spenlehauer, Gilles
10 Place Ovale
F-94230 Cachan (FR)**
Inventeur : **Bazile, Didier
15 bis rue Chevalier
F-94210 La Varenne St. Hilaire (FR)**
Inventeur : **Veillard, Michel
12 rue du Docteur Roux
F-92330 Sceaux (FR)**
Inventeur : **Prud'Homme, Christian
19 rue Commandant Faurax
F-69006 Lyon (FR)**
Inventeur : **Michalon, Jean-Paul
52 rue St. Mathieu
F-69008 Lyon (FR)**

(74) Mandataire : **Le Pennec, Magali et al
RHONE-POULENC RORER SA, Direction des Brevets, 20 Avenue Raymond Aron
F-92165 Antony Cédex (FR)**

(54) **Procédé de préparation de nanoparticules.**

(57) La présente invention concerne un procédé de préparation de nanoparticules par solubilisation d'un copolymère polylactique polyoxyde d'éthylène et/ou de propylène dans un solvant organique puis formation de nanoparticules par mélange de la solution contenant le polymère avec une solution aqueuse par précipitation sans utiliser d'agent protecteur colloïdal additionnel ou par microfluidisation et évaporation de solvant.

EP 0 520 889 A1

La présente invention concerne un nouveau procédé de préparation de particules sphériques de petites dimensions, souvent inférieures à 500 nm capables de transporter ou de vectoriser un principe actif. Ces particules, encore appelées nanoparticules, présentent l'avantage de pouvoir circuler dans le flux sanguin sans problème de dimension au niveau des capillaires. L'invention vise aussi les nouvelles particules obtenues et leur utilisation en pharmacie humaine ou animale.

Il est connu d'après l'art antérieur décrit dans le brevet FR 2 608 988 de préparer des particules de dimension inférieure à 500 nm par au moins trois types de procédé.

Le premier type de procédé consiste à effectuer une polymérisation d'un monomère dans une solution de façon à obtenir une dispersion micellaire du polymère dans la solution. Ce type de procédé est limité à des monomères polymérisables en solution, il nécessite l'élimination après l'étape de polymérisation du catalyseur de polymérisation, des oligomères de bas poids moléculaire, des monomères et des agents surfactants nécessaires à la polymérisation. Le polymère obtenu présente une répartition de poids moléculaire aléatoire.

Le deuxième et le troisième types de procédé consistent à utiliser des polymères préformés, à les solubiliser dans un solvant, à former un précipité ou une dispersion à partir d'une solution de ces polymères et d'un non solvant puis à évaporer le solvant de façon à récupérer les nanoparticules sous forme d'une suspension colloïdale. La solution solvante est généralement une solution organique du polymère, la solution non solvante est souvent une solution aqueuse.

Selon le deuxième type de procédé, le polymère est solubilisé dans un solvant organique miscible à l'eau. Lorsque cette solution est mélangée à la phase aqueuse, le polymère insoluble dans le mélange phase aqueuse/solvant organique précipite sous forme de nanoparticules.

Selon le troisième type de procédé, on émulsionne un solvant organique contenant le polymère, non miscible à l'eau, dans une phase aqueuse puis on évapore le solvant organique.

La formation du précipité ou de l'émulsion exige la présence d'une quantité d'agent surfactant loin d'être négligeable. Or lors de l'évaporation subséquente, il est très difficile d'éliminer l'agent surfactant qui subsiste dans la suspension colloïdale de nanoparticules obtenue, cet agent est souvent indésirable pour une bonne biocompatibilé. Ces deux dernières techniques ne sont donc pas utilisables pour la préparation de nanoparticules biocompatibles à cause de la présence de l'agent protecteur colloïdal.

La demande de brevet précitée FR 2 608 988 concerne un procédé de préparation de systèmes colloïdaux dispersibles sous forme de nanoparticules inférieures à 500 nm. Ces nanoparticules à base d'une substance qui peut indifféremment être un polymère et/ou un principe actif, sont obtenues par la deuxième méthode évoquée. Les nanoparticules qui sont obtenues à base d'un polymère polylactique contiennent dans la majorité des exemples une quantité d'agent tensioactif égale à la quantité de polymère. Dans cette demande dans un seul exemple (l'exemple 4), l'inventeur prétend obtenir des nanoparticules de polymère polylactique sans agent tensioactif. Nous avons reproduit cet essai, avons obtenu des nanoparticules de polymère polylactique à partir d'une solution acétonique d'acide polylactique et d'eau avec des rendements extrêmement faibles, toujours inférieurs à 10 %.. Cette technique n'est donc pas utilisable pour la préparation de nanoparticules d'acide polylactique en l'absence d'agent tensioactif.

La présente invention a permis d'obtenir des nanoparticules de polymères comportant une majorité de motifs dégradables en l'absence d'agent tensioactif. Elle consiste à préparer des nanoparticules à partir de copolymère polylactique polyoxyde d'éthylène et/ou polyoxyde de propylène en l'absence d'agent tensioactif ou d'agent protecteur colloïdal additionnel.

Elle consiste à utiliser un copolymère comportant une majorité de motifs polylactiques modifiés par incorporation de motifs polyoxyde d'éthylène et/ou polyoxyde de propylène. Ce copolymère répond pour la majorité de ses motifs de préférence à la formule (I) suivante :

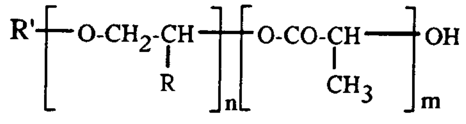

(I)

dans laquelle:

R représente dans chacun des motifs oxyde d'alkylène un groupe identique ou différent choisi parmi l'hydrogène ou un groupe méthyle

n est un nombre entier compris entre 20 et 1000

R′ représente l'hydrogène ou un motif alkyle contenant 1 à 4 atomes de carbone, de préférence un groupe méthyle

m est un nombre entier compris entre 10 et 1500.

Le motif polylactique de ce copolymère a de préférence un poids moléculaire compris entre 700 et 100

000, le motif polyoxyde d'éthylène et/ou polyoxyde de propylène a lui de préférence un poids moléculaire compris entre 1 000 et 40 000. D'une façon encore plus préférentielle le motif polymérique polylactique a un poids moléculaire compris entre 1 000 et 60 000, le motif polyoxyde d'éthylène et/ou polyoxyde de propylène a un poids moléculaire compris entre 1 000 et 6 000.

Selon une dernière préférence le polymère polylactique est un polymère contenant 50 % de motifs lactique de configuration D ($PLA_{50}$) et le polyoxyde est un polyoxyde d'éthylène.

Ces polymères se présentent de préférence sous une forme dibloc, c'est à dire que selon une manière pratique de mise en oeuvre on part d'un polyéthylène et/ou polypropylène glycol commercial monofonctionnel dans lequel R′ représente de préférence un groupe méthyle de poids moléculaire désiré c'est à dire d'un poids moléculaire compris entre 1000 et 40 000 ou encore comportant environ 20 à 1000 motifs oxyde d'éthylène ou de propylène, de préférence comportant 20 à 150 motifs oxyde d'éthylène ou 20 à 100 motifs oxyde de propylène sur lequel on greffe des motifs lactides jusqu'à obtenir le poids moléculaire désiré sur la chaîne polylactique en présence d'un catalyseur tel que notamment l'octoate d'étain.

On peut préciser que pour obtenir des blocs polylactiques d'un poids moléculaire compris entre 1 000 et 60 000, il est souhaitable d'introduire entre environ 10 et 1000 motifs lactide. On préfère tout particulièrement utiliser des copolymères polyoxyde d'éthylène et/ou de propylène polylactique dont la chaîne contient entre 10 et 150 motifs lactiques.

On préfère encore plus particulièrement partir d'un polyéthylène glycol commercial comportant 48 motifs oxyde d'éthylène d'un poids moléculaire de 2100 que l'on fait réagir avec 40 à 150 motifs lactide.

Après polymérisation en solution, les polymères obtenus sont purifiés de façon à éliminer totalement le catalyseur et le lactide subsistant. Cette purification est effectuée par précipitation du polymère à l'aide d'un non solvant ou par chromatographie d'exclusion sur gel. Le copolymère est ensuite séché et conservé sous une forme pulvérulente.

Selon un premier procédé de préparation des nanoparticules selon l'invention, le polymère polylactique-polyoxyde d'éthylène et/ou de propylène désiré est mis en solution dans un solvant ou dans un mélange de solvants, puis la solution organique est versée dans une solution aqueuse, de façon à provoquer la formation par précipitation des nanoparticules. Dans ce procédé, on n'utilise pas d'agent protecteur colloïdal additionnel. On entend par agent colloïdal, les agents dont font partie les agents tensioactifs qui favorisent la formation de colloïdes.

Le solvant ou le mélange de solvants solubilisant le copolymère est choisi parmi les cétones telle que l'acétone, les éthers cycliques tels que le tétrahydrofurane, les dioxanes; les nitriles tels que l'acétonitrile. On préfère utiliser l'acétone. La solubilité du copolymère dans ces solvants doit être d'au moins 10 g/l.

Le rapport volumique entre la solution aqueuse et la solution du copolymère est compris de préférence entre 0,5 et 10, et tout particulièrement entre 1 et 10. La quantité de copolymère introduite dans le solvant dépend bien entendu de sa solubilité, mais pour une meilleure mise en oeuvre de l'invention, c'est à dire essentiellement pour obtenir un rendement optimum en nanoparticules formées, une quantité comprise entre 10 et 50 mg/ml est préférée.

Selon un deuxième procédé de préparation des nanoparticules, le pôlymère polylactique-polyoxyde d'éthylène et/ou de propylène est mis en solution dans un ester de préférence dans l'acétate d'éthyle puis la solution organique est versée dans la solution aqueuse. Les nanoparticules sont formées par l'utilisation d'un microfluidiseur.

Le solvant du copolymère est ensuite évaporé en chauffant la solution colloïdale de nanoparticules au dessus de la température d'ébullition du solvant dans le cas où l'élimination est effectuée à pression atmosphérique ou à une température inférieure si l'évaporation est effectuée sous pression réduite. Après que le solvant ait été éliminé, la suspension de nanoparticules dans l'eau est filtrée sur un filtre de diamètre de pores d'environ 1 μm, de façon à éliminer les agrégats et les grosses particules. Le rendement en nanoparticules obtenues dépasse généralement 50 %.

La formation de nanoparticules peut être effectuée en présence d'un principe actif pharmaceutique qui peut être introduit soit dans le solvant du copolymère soit dans le solvant de précipitation, il doit de préférence être soluble dans le solvant du polymère et non soluble dans l'eau, bien qu'il soit toujours possible de former des nanoparticules si le principe actif est soluble dans l'eau, le rendement en sera néanmoins amoindri.

Les nanoparticules obtenues qui font également partie de la présente invention ne contiennent que le polymère de formule (I) et éventuellement un principe actif si la précipitation est effectuée en présence d'un principe actif. Elles présentent un diamètre moyen compris entre 50 et 500 nm et de préférence un diamètre moyen compris entre 50 et 250 nm.

Les nanoparticules obtenues sont utilisées dans de nombreux domaines tels que l'agrochimie, le papier pour reprographie, le papier photographique, mais de part leur nature parfaitement biocompatible ces nanoparticules sont plus particulièrement destinées à l'industrie pharmaceutique humaine ou animale. Ces produits

sont injectables par voie intramusculaire, sous cutanée, intraartérielle, intraveineuse, intraorgane ou intracavités sans risque anaphylactique

L'invention sera plus complètement décrite à l'aide des exemples suivants qui ne doivent pas être considérés comme limitatifs de l'invention.

EXEMPLE 1

Préparation de copolymères polylactique polyéthylène glycol

1.1) polymère $PLA_{50}^{2900}$-$PEG^{2100}$

Dans un ballon tricol de 250 ml muni d'un agitateur à palettes et d'un réfrigérant ascendant avec circulation d'azote sec, le ballon étant chauffé par un bain d'huile régulé, on introduit :

| | |
|---|---|
| dl lactide | 144 g |
| polyéthylène glycol | 79.3 g |
| octoate stanneux | 0.256 g |
| toluène distillé | 335 g |

Le lactide est recristallisé la veille dans l'acétate d'éthyle, puis lavé le jour même à l'éther éthylique. On le séche sous vide. On charge tous les réactifs puis on chauffe sous léger reflux (110-114°C) pendant 5 heures et demi. Le solvant est ensuite éliminé sous vide à l'aide d'un évaporateur rotatif (40 mm de Hg - 100°C).

On obtient 226.3 g de concentrat.

La purification du copolymère est effectuée de la façon suivante:

On charge:

| | |
|---|---|
| concentrat | 215 g |
| dichlorométhane | 280 g |

On agite jusqu'à formation d'une solution homogène. Cette solution est coulée lentement dans 900 ml d'hexane à froid. Le polymère précipite sous forme d'une pâte qui est séparée par décantation. Le catalyseur de polymérisation est éliminé dans la phase hexanique. Aprés séparation du polymère il est mis à sécher dans une étuve sous vide à 40 °C. On obtient 188.4 g de copolymère dont la masse est analysée par résonnance magnétique nucléaire, la masse de polyéthylène glycol est de 2100 et celle de polylactique de 2900, ce qui représente 40 motifs lactique et 48 motifs oxyde d'éthylène

1.2) polymère $PLA_{50}^{9600}$-$PEG^{2100}$

On reproduit l'exemple 1.1 en introduisant les composés suivants:

| | |
|---|---|
| dl lactide | 48.6 g |
| polyéthylène glycol | 10 g |
| octoate stanneux | 0.085 g |
| toluène distillé | 90 g |

après réaction on obtient 63,6 g de concentrat que l'on purifie par la méthode suivante : on met en solution 40 g de concentrat dans 200 g de dichlorométhane jusqu'à l'obtention d'une solution homogène. Cette solution est versée lentement dans 800 ml d'eau maintenu entre 55 et 60°C. Le polymère précipite et le dichlorométhane est évaporé, le lactide non réagi reste en solution aqueuse, le polymère est centrifugé puis séché à l'étuve sous vide à 40°C.

On obtient 35g de polymère dont l'analyse par résonnance magnétique nucléaire permet de déterminer le poids moléculaire. Ce dernier est de 9600 pour la chaîne lactique et de 2100 pour la chaîne polyoxyde d'éthylène, ce qui représente 133 motifs lactique et 48 motifs oxyde d'éthylène.

1.3) polymère $PLA_{50}^{40000}$

Dans un réacteur d'un litre chauffé par un bain d'huile et muni d'un agitateur sous forme d'une ancre et d'un réfrigérant ascendant et maintenu sous azote, on introduit 180 g de xylène distillé avant emploi et 0,180 g d'octoate d'étain, on chauffe, puis on introduit 120 g de dl lactide S de la société Boehringer préalablement recristallisé dans l'acétate d'éthyle et lavé à l'éther sulfurique.

On laisse réagir 5 heures à 140°C , en fin de réaction, on refroidit rapidement puis on élimine une partie du xylène sous vide. On dissout le polymère dans le dichlorométhane et on le précipite par le méthanol. On le sèche dans une étuve à vide à 65°C.

EXEMPLE 2

Préparation de nanoparticules à partir de ces polymères

On utilise 50 mg du copolymère préparé en 1.1 que l'on dissout dans 0,5; 2,5; 5 et 10 ml d'acétone. Les nanoparticules sont préparées par précipitation en versant lentement ce volume dans 5 ml d'eau. La suspension colloïdale obtenue est évaporée 30 minutes au rotavapor à température ambiante et sous une pression de 3 mm de mercure. La suspension est alors filtrée sur un filtre de 1,2 µm afin d'éliminer les grosses particules et les aggrégats. Le diamètre des particules ainsi que le rendement d'obtention des particules sur le polymère engagé sont indiqués dans le tableau ci-joint.

| Eau/acétone | 10/1 | 2/1 | 1/1 | 0,5/1 |
|---|---|---|---|---|
| Rendement | 75 % | 76 % | 92 % | 79 % |
| Diamètre (nm) | 67±36 | 63±30 | 50±21 | 38±10 |

EXEMPLE COMPARATIF SELON L'EXEMPLE 4 DE FR 2 608 988

On utilise le même protocole mais avec le polymère lactique de l'exemple 1.3 de poids moléculaire 40 000. Les nanoparticules sont préparées par précipitation en versant la solution acétonique dans 5 ml d'eau. L'acétone est évaporée au rotavapor pendant 30 minutes. Pendant cette phase le polymère précipite et se colle sur les parois. La suspension est recueillie, filtrée sur un filtre de 1,2 µm de diamètre. Le filtrat est totalement limpide. La concentration en polymère est trop faible pour être mesurable.

EXEMPLE 3

Préparation de nanoparticules contenant un principe actif.

On dissout dans 2 ml d'acétone 40 mg de spiramycine et 200 mg du copolymère préparé selon le point 1.2 ci-dessus. Les nanoparticules sont préparées par précipitation en versant la solution acétonique dans 20 ml de tampon phosphate 0,1 M, pH 7,4.

L'acétone est évaporée au rotavapor pendant 30 minutes puis la solution est filtrée sur un filtre de 1,2 µm. On obtient des nanoparticules de spiramycine et de copolymère d'un diamètre de 74 nm.

EXEMPLE 4

Préparation de nanoparticules par microfluidisation.

Sans agent tensio-actif.

Le polymère (100 mg) est solubilisé dans 1 ml d'acétate d'éthyle. Cette solution organique est versée dans 10 ml d'eau sous agitation à l'ultraturrax. Après 30 secondes d'agitation, l'émulsion est homogénéisée et affinée pendant 2 minutes par recyclage à l'aide d'un microfluidiseur (MICROFLUIDICS 110 S). L'acétate d'éthyle est éliminé par évaporation sous vide partiel pendant 45 minutes, puis les nanoparticules sont filtrées à travers un filtre SARTORIUS ou MILLIPORE de porosité 1.2 µm.

| ESSAIS | NATURE DU POLYMERE PLA - PEG | TAILLE (nm) | RENDEMENT (%) |
|---|---|---|---|
| 1 | 2900/2100 | 65 | 71 |
| 2 | 9600/2100 | 100 | 85 |
| 3 | 8000/4000 | 60 | 87 |
| 4 | 16000/4000 | 120 | 61,2 |
| 5 | 30000/2000 | 280 | N.D |
| 6 | 30000/5000 | 150 | 65,5 |
| C1 | 52000/0 | INFAISABLE PRECIPITATION INSTANTANEE DU POLYMERE | |

N.B. : les polymères 8000/4000, 16000/4000, 30000/2000 et 30000/5000 sont préparés de la même manière qu'à l'exemple 1 à partir des quantités suivantes de polyéthylène glycol, de DL lactide, de toluène et d'octoate d'étain.

| ESSAIS | PEG | | DL LACTIDE | TOLUENE | OCTOATE D'ETAIN |
|---|---|---|---|---|---|
| | NATURE | POIDS | | | |
| 3 | 4000 | 13 g | 26 g | 40 g | 0,05 |
| 4 | 4000 | 10 g | 40 g | 60 g | 0,08 g |
| 5 | 2000 | 2,5 g | 37,5 g | 60 g | 0,08 g |
| 6 | 5000 (méthyl ester) | 5 g | 30 g | 45 g | 0,06 g |

**Revendications**

- 1 - Procédé de préparation de nanoparticules caractérisé en ce que on solubilise un copolymère polylactique polyoxyde d'éthylène et/ou polyoxyde de propylène comportant une majorité de motifs de formule:

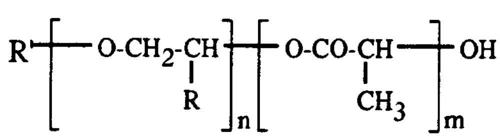

(I)

dans laquelle:

R représente dans chacun des motifs oxyde d'alkylène un groupe identique ou différent choisi parmi

l'hydrogène ou un groupe méthyle

R′ représente l'hydrogène ou un motif alkyle contenant 1 à 4 atomes de carbone

n est un nombre entier compris entre 20 et 1000

m est un nombre entier compris entre 10 et 1500.

dans un solvant organique puis on forme les nanoparticules par mélange de la solution contenant le polymère avec une solution aqueuse sans utiliser d'agent protecteur colloïdal additionnel.

**- 2 -** Procédé selon la revendication 1 caractérisé en ce que dans la formule (I) n est compris entre 20 et 150 et m est compris entre 10 et 1000.

**- 3 -** Procédé selon la revendication 2 caractérisé en ce que dans la formule (I) R représente un groupe méthyle, R' représente un groupe méthyle et n est compris entre 20 et 100.

**- 4 -** Procédé selon la revendication 3 caractérisé en ce que dans la formule (I) R représente l'atome d'hydrogène,

R′ représente un groupe méthyle

n est égal à 48

m est compris entre 40 et 150.

**- 5 -** Procédé selon la revendication 1 caractérisé en ce que le solvant du polymère polyoxyde d'éthylène et/ou polyoxyde de propylène polylactique est choisi parmi les cétones, les éthers, les dioxanes, les nitriles.

**- 6 -** Procédé selon la revendication 5 caractérisé en ce que le solvant est l'acétone.

**- 7 -** Procédé selon la revendication 1 caractérisé en ce que le rapport volumique entre la solution aqueuse et la solution organique du copolymère est compris entre 0,5 et 10.

**- 8 -** Procédé selon la revendication 1 caractérisé en ce que la concentration du copolymère dans la solution organique est supérieure ou égale à 10 g/l.

**- 9 -** Procédé selon l'une quelconque des revendications précédentes caractérisé en ce que on ajoute à l'une des solutions un principe actif pharmaceutique.

**- 10 -** Nanoparticules caractérisées en ce qu'elles sont constituées majoritairement d'un copolymère de formule:

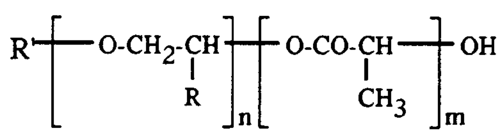

(I)

dans laquelle:

R représente dans chacun des motifs oxyde d'alkylène un groupe identique ou différent choisi parmi l'hydrogène ou un groupe méthyle

R′ représente l'hydrogène ou un motif alkyle contenant 1 à 4 atomes de carbone

n est un nombre entier compris entre entre 20 et 1000

m est un nombre entier compris entre 10 et 1500 et qu'elles ne contiennent pas d'agent colloïdal.

**- 11 -** Nanoparticules selon la revendication 10 caractérisées en ce qu'elles présentent une dimension moyenne comprise entre 50 et 500 nm

**- 12 -** Nanoparticules selon la revendication 11 caractérisées en ce qu'elles présentent une dimension moyenne comprise entre 50 et 250 nm.

Office européen des brevets

# RAPPORT DE RECHERCHE EUROPEENNE

Numero de la demande

EP 92 40 1789

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 166 596 (ICI PLC) | 1,2,4, 7-10 | C08J3/14<br>C08G63/66<br>A61K9/51<br>//C08L67/04, 71:02 |
| Y | * exemples 1,4-6 *<br>* page 9, ligne 26 - page 10, ligne 9 *<br>* revendications 1,2,5 *<br>--- | 1,2,4-12 | |
| D,Y | EP-A-0 275 796 (CENTRE NATIONAL DE LA RECHERCHE SCIENTIFIQUE)<br>* revendications 1,6-10,15 *<br>* colonne 3, ligne 33 - colonne 4, ligne 2 *<br>* colonne 4, ligne 34 - colonne 5, ligne 8 *<br>* colonne 5, ligne 27 - ligne 31 *<br>----- | 1,2,4-12 | |

| DOMAINES TECHNIQUES RECHERCHES (Int. Cl.5) |
|---|
| C08J<br>C08G<br>A61K |

Le présent rapport a été établi pour toutes les revendications

| Lieu de la recherche | Date d'achèvement de la recherche | Examinateur |
|---|---|---|
| LA HAYE | 31 AOUT 1992 | NIAOUNAKIS M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)